# EUROPEAN PATENT APPLICATION

(11) **EP 4 209 197 A1**
(43) Date of publication of application: **12.07.2023**
(21) Application number: 21863417.8
(22) Date of filing: 27.07.2021
(51) Int. Cl.: A61F 2/24

(54) **IMPLANT LOADING TOOL AND MEDICAL DEVICE**

(30) Priority: 02.09.2020 CN 202010912005
(71) Applicant: Shanghai MicroPort CardioFlow Medtech Co., Ltd., Shanghai 201203 (CN)
(72) Inventor: TAO, Lin, Shanghai 201203 (CN); ZHANG, Jingyi, Shanghai 201203 (CN); GUI, Baozhu, Shanghai 201203 (CN); CHEN, Guoming, Shanghai 201203 (CN)
(74) Representative: Lavoix
(86) International application number: PCT/CN2021/108774
(87) International publication number: WO 2022/048349

(57) **Abstract**

An implant loading tool and a medical device are disclosed. The implant loading tool includes an outflow cone (10) and an inflow cone (20) configured to detachably coupled to the outflow cone (10). The inflow cone (20) is configured to be inserted into the outflow cone (10) in a first direction, thereby performing a first compression against an implant. The inflow cone (20) is also configured to be detachably coupled to the outflow cone (10) in a second direction opposite to the first direction, thereby performing a second compression against the implant. When the inflow cone (20) is coupled to the outflow cone (10) in the second direction, it is coaxial with the outflow cone (10) and is prevented from radial and axial displacement relative to the outflow cone (10). In this way, the inflow cone (20) is cleverly used to compress the implant twice in opposite directions, entailing a simpler loading tool structure and enabling the inflow cone (20) and the outflow cone (10) to be maintained in coaxiality during the second compression. This reduces an operator's operational difficulty and makes the whole crimping process easier and more efficient.

## Description

### TECHNICAL FIELD

The present invention relates to the field of medical instruments and, in particular, to implant loading tools and a medical device.

### BACKGROUND

Interventional aortic valve implantation is a novel minimally invasive valve replacement technique that has been developed abroad in recent years, which involves loading a prosthetic valve in a delivery system and delivering it through a catheter to the aortic root, where a stent is released to ensure that the valve is anchored to the aortic valve annulus, thus functionally replacing the dysfunctional native valve and improving the patient's cardiac condition. This technique is able to treat aortic valve disease without surgically opening the chest or stopping the heartbeat as is conventional, which may cause significant trauma to the patient.

This technique requires the stent to be compressed to a diameter that is small enough to allow it to be loaded in the catheter of the delivery device. However, the stent or the valve on the stent is prone to damage or breakage due to excessive or uneven compression or accidental local flexing, which may impair the functionality or shorten the service life of the stent or valve, or even make the implantation or normal operation thereof impossible. The anchoring and compression of a self-expanding stent is particularly challenging because it will undergo tension, making the stent more prone to damage or breakage during loading. This on the one hand imposes strict requirements on the operator and, on the other hand, leads to a prolonged surgical procedure and a higher surgical risk.

Loading a valve prosthesis into a loading tool typically involves initial compression of a stent as a result of cooperation of an outflow cone with an inflow cone and secondary compression of an inflow section of the stent by a lumen of the inflow cone until the valve prosthesis is fully crimped. Extensive tests have proven that coaxiality of a stent with a loading tool is crucial to even crimping of the stent. Existing loading tools rely on the operator's manual control for maintaining such coaxiality. This places strict requirements on the operator. Although operators may receive training on interventional aortic valve implantation, it has been found in clinical practice that since such surgical procedures are complex and involve many steps, and as stent crimping accounts for only a very a small part thereof, this part of operation is often neglected or associated with insufficient training. As a result, inappropriate stent crimping often occurs, which may affect the loading or even use of a stent.

Therefore, there is a need for an easy-to-use loading tools capable of efficient loading.

### SUMMARY OF THE INVENTION

It is an object of the present invention to solve the problem that existing loading tools rely on manual operation for maintaining coaxiality in crimping through presenting implant loading tools and a medical device.

The above object is attained by an implant loading tool provided in the present invention, which comprises an outflow cone and an inflow cone configured to be detachably coupled to the outflow cone, wherein the outflow cone has, along an axis thereof, a first site and a second site that is opposite to the first site, the outflow cone configured to be coupled to the inflow cone in a direction from the second site to the first site, the inflow cone having, along an axis thereof, a third site and a fourth site that is opposite to the third site,
the inflow cone configured to be inserted into the outflow cone in a first direction from the third site to the fourth site to perform a first compression against an implant, the inflow cone also configured to be detachably coupled to the outflow cone in a second direction from the fourth site to the third site to perform a second compression against the implant,
wherein when the inflow cone is coupled to the outflow cone along the second direction, it is coaxial with the outflow cone and is prevented from radial displacement and axial displacement relative to the outflow cone.

Optionally, the outflow cone may comprise a first snap fastener and a fourth snap fastener, and the inflow cone may comprise a second snap fastener, a fifth snap fastener and a third snap fastener,
wherein when the inflow cone is inserted into the outflow cone along the first direction, the first snap fastener snap engages with the second snap fastener, thereby preventing the outflow cone from radial displacement and axial displacement with respect to the inflow cone and performing the first compression against the implant, wherein when the inflow cone is further inserted into the outflow cone along the first direction after the snap engagement of the first snap fastener with the second snap fastener, the fourth snap fastener snap engages with the fifth snap fastener, thereby preventing the outflow cone from radial displacement, axial displacement and circumferential rotation with respect to the inflow cone and performing a third compression against the implant,
wherein when the inflow cone is detachably coupled to the outflow cone in the second direction, the first snap fastener and/or the fourth snap fastener snap engages with the third snap fastener, thereby preventing the outflow cone from radial displacement and axial displacement with respect to the inflow cone and performing the second compression against the implant.

Optionally, the first snap fastener may comprise at least two first snap elements, wherein the fourth snap fastener comprises at least two second snap elements, and wherein the outflow cone comprises a plurality of cutout slots extending axially, the cutout slots being open at the first site, wherein the first snap elements and the second snap elements are both distributed circumferentially around the outflow cone, wherein the cutout slots are arranged on both circumferential sides of each first snap element, and wherein the cutout slots are arranged on both circumferential sides of each second snap element.

Optionally, each first snap element may have a first snap surface facing the second site and a first engagement surface facing the interior of the outflow cone, wherein the second snap fastener has a first bevel inwardly-tapered toward the fourth site, second snap surfaces facing the third site and a second engagement surface facing the exterior of the inflow cone, wherein the first bevel is configured to abut against the first engagement surface and to push a free end of the first snap element to deflect outwardly, wherein the first engagement surface is configured to abut against the second engagement surface and thereby preventing the outflow cone from radial displacement relative to the inflow cone, and wherein the first snap surface is configured to abut against the second snap surface and thereby preventing the outflow cone from axial displacement toward the second site relative to the inflow cone.

Optionally, the third snap fastener may comprise snap pockets, wherein a cross-sectional shape of the third snap fastener along an axis of the inflow cone matches a cross-sectional shape of each first snap element along an axis of the outflow cone, wherein the first snap element is configured to snap engage into the corresponding snap pocket and thereby preventing the inflow cone from radial and axial displacement relative to the outflow cone.

Optionally, a spacing area is present between adjacent snap pockets, wherein a circumferential length of the spacing area is not smaller than a circumferential length of each first snap element along the outflow cone, wherein the spacing areas is configured for insertion of the first snap element therein toward the fourth site, wherein the first snap elements are configured to circumferentially move into snap engagement with the snap pockets as a result of a relative circumferential rotation of the outflow cone and the inflow cone.

Optionally, a side wall of each snap pocket facing the interior of the inflow cone may flare outwardly toward the third site.

Optionally, each second snap element may have a second bevel facing the exterior of the outflow cone and a third snap surface facing the second site, wherein the second bevel is tapered inwardly toward the first site, wherein the fifth snap fastener has a fourth snap surface facing the fourth site, a first stop surface facing the interior of the inflow cone and two second stop surfaces arranged in opposition to each other along a circumference of the inflow cone, wherein the first stop surfaces is configured to abut against the second bevel so as to push a free end of the second snap element to deflect toward the interior of the outflow cone, wherein the first stop surface is configured to abut against an external side wall of the outflow cone and thereby preventing the outflow cone from radial displacement relative to the inflow cone, wherein the third snap surface is configured to abut against the fourth snap surface and thereby preventing the outflow cone from axial displacement toward the second site relative to the inflow cone, wherein the second stop surfaces are configured to abut against radial side faces of the second snap elements and thereby preventing the outflow cone from circumferential rotation relative to the inflow cone.

Optionally, a maximum axial distance from the first snap fastener to the second site may be not smaller than a maximum axial distance from the fourth snap fastener to the second site.

Optionally, the third snap fastener may comprise snap plates each arranged along an axial direction of the inflow cone, wherein each snap plate has a snap opening extending therethrough in the axial direction of the inflow cone, wherein the second snap element is configured to snap engage into the snap opening, and wherein the third snap surface of the second snap element facing the second site is configured to abut against a side of the snap plate facing the fourth site and thereby preventing the inflow cone from radial displacement and axial displacement relative to the outflow cone.

Optionally, the outflow cone may comprise a sixth snap fastener and the inflow cone may comprise a third snap fastener,
when the inflow cone is detachably coupled to the outflow cone in the second direction, the sixth snap fastener snap engages with the third snap fastener, thereby preventing the outflow cone from radial displacement and axial displacement relative to the inflow cone.

Optionally, the sixth snap fastener may comprise at least two third snap elements and the outflow cone may comprise a plurality of cutout slots extending axially, the cutout slots being open at the first site, wherein the third snap elements are distributed circumferentially around the outflow cone and the cutout slots are arranged on both circumferential sides of each third snap element, wherein each third snap element has a third bevel facing the exterior of the outflow cone and a fifth snap surface facing the second site, the third bevel inwardly tapered toward the first site.

Optionally, the third snap fastener may comprise snap plates each arranged along an axial direction of the inflow cone, wherein each snap plate has a snap opening extending therethrough in the axial direction of the inflow cone, wherein the third snap element is configured to snap engage into the snap opening, and wherein the fifth snap surface is configured to abut against a side of the snap plate facing the fourth site and thereby preventing the inflow cone from radial displacement and axial displacement relative to the outflow cone.

Optionally, the implant loading tool may further comprise a securing member including an internal bore axially extending therethrough, wherein the securing member is configured to be detachably coupled to the second site; wherein when the securing member is coupled to the second site, the internal bore is coaxial with the outflow cone and the securing member is prevented from radial displacement and axial displacement relative to the outflow cone.

Optionally, the securing member may be coupled to the outflow cone by threads, snap means or a pin.

Optionally, the implant loading tool may further comprise a capsule guide tube, wherein the capsule guide tube is configured to be detachably inserted in the internal bore, wherein the capsule guide tube is coupled to the securing member, the capsule guide tube is coaxial with the internal bore and is prevented from radial displacement and axial displacement relative to the securing member when coupled to the securing member.

Optionally, an inner diameter of the internal bore may match an outer diameter of the capsule guide tube, wherein the capsule guide tube is configured to be inserted in the internal bore and is prevented from radial displacement relative to the securing member.

Optionally, the capsule guide tube may have, along an axis thereof, a fifth site and a sixth site that is opposite to the fifth site and is configured to be coupled to the securing member in a direction from the sixth site to the fifth site, wherein the securing member has, along an axis thereof, a seventh site and an eighth site that is opposite to the seventh site, wherein the capsule guide tube is configured to be coupled to the securing member in a direction from the eighth site to the seventh site, wherein the capsule guide tube has a third stop surface facing the sixth site, wherein the securing member has a fourth stop surface facing the seventh site, the third stop surface configured to abut against the fourth stop surface and thereby preventing the capsule guide tube from axial displacement toward the sixth site relative to the securing member.

The above object is also attained by a medical device provided in the present invention, which comprises the implant loading tool as defined above and a delivery device. The loading tool is configured to work with the delivery device to load an implant into the delivery device.

The above object is also attained by another implant loading tool provided in the present invention, which comprises an outflow cone and an inflow cone configured to be detachably coupled to the outflow cone. At least one of the outflow cone and the inflow cone is provided with a snap fastener. The inflow cone has, along an axis thereof, a third site and a fourth site that is opposite to the third site and wherein when the inflow cone is inserted into the outflow cone in a first direction from the third site to the fourth site, the inflow cone is configured to apply a first compressive force to the implant. When the inflow cone is detachably coupled to the outflow cone in a second direction from the fourth site to the third site, the inflow cone is configured to apply a second compressive force to the implant. During the application of the first or second compressive force, the snap fastener is configured to limit the inflow cone so as to bring the inflow cone into coaxiality with the outflow cone.

In summary, the present invention provides an implant loading tool and a medical device. The implant loading tool includes an outflow cone and an inflow cone configured to be detachably coupled to the outflow cone. The inflow cone is configured to be inserted into the outflow cone in a first direction, so as to perform a first compression against an implant. The inflow cone is also configured to be detachably coupled to the outflow cone in a second direction opposite to the first direction, to perform a second compression against the implant. The inflow cone is so coupled to the outflow cone in the second direction so as to be coaxial with the outflow cone and is prevented from radial displacement and axial displacement relative to the outflow cone.

With this arrangement, the first compression of the implant is first performed by the outflow cone and the inflow cone, and the inflow cone is then flipped over and coupled to the outflow cone to perform the second compression against the implant. During the second compression, the inflow cone is kept coaxial with the outflow cone and is prevented from radial displacement and axial displacement relative to outflow cone. In this way, the inflow cone is cleverly used to compress the implant twice in opposite directions, entailing a simpler loading tool structure and enabling the inflow cone and the outflow cone to be maintained in coaxiality during the second compression. This ensures coaxial crimping of the implant during the second compression and straightness throughout the entire operation. As a result, an operator is enabled to more easily accomplish the second compression, and the whole crimping process is made easier and more efficient.

### BRIEF DESCRIPTION OF THE DRAWINGS

As would be appreciated by those of ordinary skill in the art, the following figures are provided to enhance an understanding of the present invention and do not limit the scope thereof in any sense, in which:
Fig. 1 is a schematic illustration of an implant loading tool according to a first embodiment of the present invention;
Fig. 2a is a schematic diagram illustrating a first snap fastener and a second snap fastener in snap engagement with each other according to the first embodiment of the present invention;
Fig. 2b is a schematic cross-sectional view of Fig. 2a taken along an axial plane;
Fig. 2c is an enlarged view of portion A of Fig. 2b;
Fig. 3a is a schematic diagram illustrating a fourth snap fastener and a fifth snap fastener in snap engagement with each other according to the first embodiment of the present invention;
Fig. 3b is a schematic cross-sectional view of Fig. 3a taken along an axial plane;
Fig. 3c is an enlarged view of portion B of Fig. 2b;
Fig. 4 is a schematic cross-sectional view taken along an axial plane, showing how a securing member, a capsule guide tube and an outflow cone are coupled together in accordance with the first embodiment of the present invention;
Fig. 5a is a schematic illustration of the outflow cone according to the first embodiment of the present invention;
Fig. 5b is a schematic cross-sectional view of the outflow cone of Fig. 5a taken along an axial plane;
Fig. 5c schematically illustrates the outflow cone of Fig. 5a viewed in the direction from a first site toward a second site;
Fig. 6a is a schematic illustration of an outflow cone according to the first embodiment of the present invention;
Fig. 6b is a schematic cross-sectional view of the inflow cone of Fig. 6a taken along an axial plane;
Fig. 6c is a perspective view of the inflow cone of Fig. 6a;
Fig. 7a is a schematic diagram illustrating the first snap fastener and a third snap fastener in snap engagement with each other according to the first embodiment of the present invention;
Fig. 7b is an enlarged view of portion C of Fig. 7a;
Fig. 7c is a schematic diagram illustrating a side wall of a snap pocket according to the first embodiment of the present invention;
Fig. 7d is an enlarged view of portion D of Fig. 7c;
Fig. 8 is a schematic illustration of an implant according to the first embodiment of the present invention;
Fig. 9 is a schematic illustration of a delivery device according to the first embodiment of the present invention;
Fig. 10a is a schematic diagram illustrating a first step in a process of using the loading tool according to the first embodiment of the present invention;
Fig. 10b is a schematic cross-sectional view of Fig. 10a taken along an axial plane;
Fig. 11a is a schematic diagram illustrating a second step in the process of using the loading tool according to the first embodiment of the present invention;
Fig. 11b is a schematic cross-sectional view of Fig. 11a taken along an axial plane;
Fig. 12a is a schematic diagram illustrating a third step in the process of using the loading tool according to the first embodiment of the present invention;
Fig. 12b is a schematic cross-sectional view of Fig. 12a taken along an axial plane;
Fig. 12c is an enlarged view of portion E of Fig. 12b;
Fig. 12d is a schematic partial cross-sectional view of the capsule guide tube of Fig. 12b taken along an axial plane;
Fig. 13a is a schematic diagram illustrating a fourth step in the process of using the loading tool according to the first embodiment of the present invention;
Fig. 13b is a schematic cross-sectional view of Fig. 13a taken along an axial plane;
Fig. 14a is a schematic illustration of an outflow cone according to a second embodiment of the present invention;
Fig. 14b is a schematic cross-sectional view of Fig. 14a taken along an axial plane;
Fig. 15a is a schematic illustration of an inflow cone according to the second embodiment of the present invention;
Fig. 15b is a schematic perspective view of Fig. 15a;
Fig. 16a is a schematic diagram illustrating a first snap fastener and a second snap fastener in snap engagement with each other according to the second embodiment of the present invention;
Fig. 16b is a schematic diagram illustrating a fourth snap fastener and a fifth snap fastener in snap engagement with each other according to the second embodiment of the present invention;
Fig. 17 is a schematic diagram illustrating the fourth snap fastener and a third snap fastener in snap engagement with each other according to the second embodiment of the present invention;
Fig. 18a is a schematic illustration of an outflow cone according to a third embodiment of the present invention;
Fig. 18b is a schematic cross-sectional view of Fig. 18a taken along an axial plane;
Fig. 19a is a schematic illustration of an inflow cone according to the third embodiment of the present invention;
Fig. 19b is a schematic perspective view of Fig. 19a;
Fig. 20a is a schematic diagram illustrating a first snap fastener and a second snap fastener in snap engagement with each other according to the third embodiment of the present invention;
Fig. 20b is a schematic diagram illustrating a fourth snap fastener and a fifth snap fastener in snap engagement with each other according to the third embodiment of the present invention;
Fig. 21a is a schematic diagram illustrating the fourth snap fastener and a third snap fastener in snap engagement with each other according to the third embodiment of the present invention;
Fig. 21b is a perspective view of the fourth snap fastener and the third snap fastener in snap engagement with each other according to the third embodiment of the present invention;
Fig. 22 is a schematic cross-sectional view of an outflow cone according to a fourth embodiment of the present invention taken along an axial plane;
Fig. 23 is a schematic cross-sectional view of an inflow cone according to the fourth embodiment of the present invention taken along an axial plane; and
Fig. 24 is a schematic partial cross-sectional view of the outflow cone and the inflow cone being coupled to each other according to the fourth embodiment of the present invention taken along an axial plane.

In these figures,
5, a delivery device; 54, a conical head; 55, an anchor; 56, a sheath;
9, a valve stent; 91, attachment projections; 92, an outflow section; 93, an inflow section;
10, an outflow cone; 101, a first site; 102, a second site; 11, a first snap fastener; 111, a first snap element; 112, a first snap surface; 113, a first engagement surface; 12, a fourth snap fastener; 121, a second snap element; 122, a second bevel; 123, a third snap surface; 13, a cutout slot; 14, a sixth snap fastener; 141, a third snap element; 142, a third bevel; 143, a fifth snap surface; 151, a first section; 152, a third section; 153, a sixth section; 154, a seventh section;
20, an inflow cone; 201, a third site; 202, a fourth site; 21, a second snap fastener; 211, a first bevel; 212, a second snap surface; 213, a second engagement surface; 22, a third snap fastener; 221, a snap pocket; 222, a side wall; 23, a fifth snap fastener; 231, a fourth snap surface; 232, a first stop surface; 233, a second stop surface; 24, a receptacle; 25, a snap plate; 251, a snap opening; 261, a second section; 262, an eighth section; 263, a fourth section; 264, a fifth section; 265, a ninth section;
30, a securing member; 301, a seventh site; 302, an eighth site;
40, a capsule guide tube; 401, a fifth site; 402, a sixth site; and 41, a third stop surface.

### DETAILED DESCRIPTION

The above and other objects, advantages and features of the present invention will become more apparent from the following detailed description of several specific embodiments thereof, which is to be read in conjunction with the accompanying drawings. It is noted that the figures are presented in a very simplified form not necessarily drawn to exact scale for the only purpose of helping to explain the disclosed embodiments in a more convenient and clearer way. In addition, structures shown in the figures are usually portions of actual structures. In particular, as the figures tend to have distinct emphases, they are often drawn to different scales.

As used herein, the singular forms "a", "an" and "the" include plural referents and the term "or" is generally employed in the sense including "and/or", "several" including "at least one" and "at least two" including "two or more". Additionally, the use of the terms "first", "second" and "third" herein is intended for illustration only and is not to be construed as denoting or implying relative importance or as implicitly indicating the numerical number of the referenced items. Accordingly, defining an item with "first", "second" or "third" is an explicit or implicit indication of the presence of one or at least two of the items. The term "proximal end" usually refers to an end closer to an operator, and the term "distal end" usually refers to an end closer to a patient, i.e., close to an object to be punctured. The terms "one end" and "the other end", or "proximal end" and "distal end", are generally used to refer to opposite end portions including the opposite endpoints, rather than only to the endpoints. As used herein, the terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two components. Furthermore, when an element is referred herein to as being disposed on another element, it is typically only meant that the two elements are connected, coupled, mated or geared to each other either directly or indirectly with the presence of intervening element(s). Such reference should not be interpreted as indicating or implying any relative spatial relationship between the two elements. That is, the referenced element may be positioned inside, outside, above, beneath, beside or in any other spatial relationship to the other element, unless the context clearly specifies. Those of ordinary skill in the art can understand the specific meanings of the above-mentioned terms herein, depending on their context.

In general, the present invention aims to provide implant loading tools and a medical device, which solve the problem that existing loading tools rely on manual operation for maintaining coaxiality in crimping.

A description is set forth below with reference to the annexed figures.

### Embodiment 1

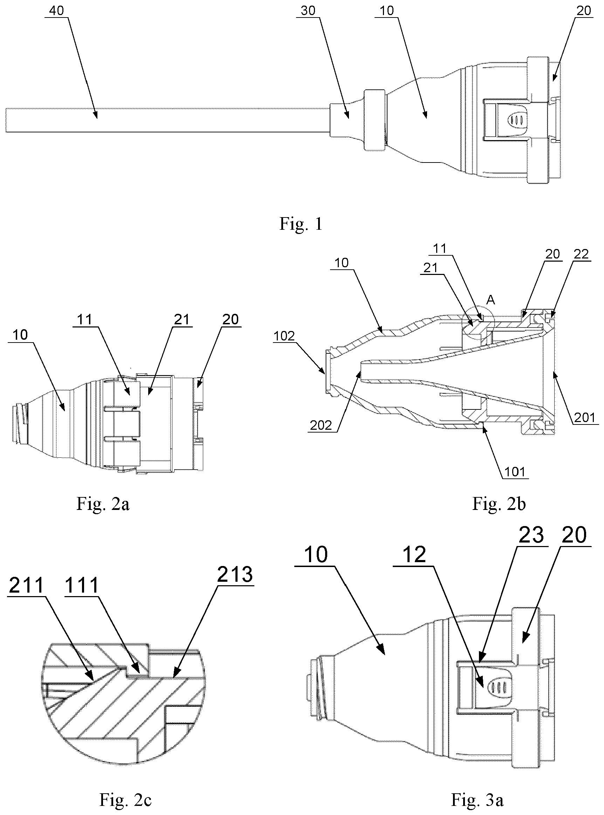

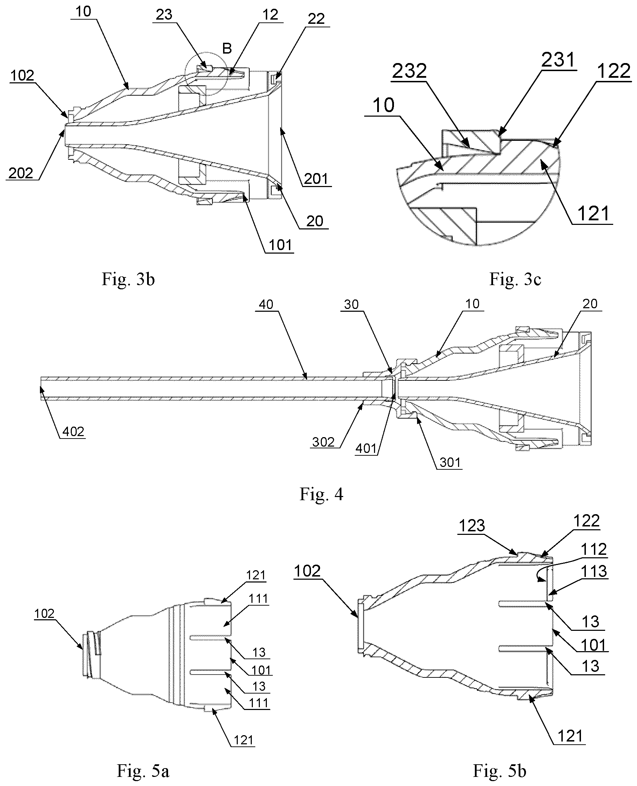

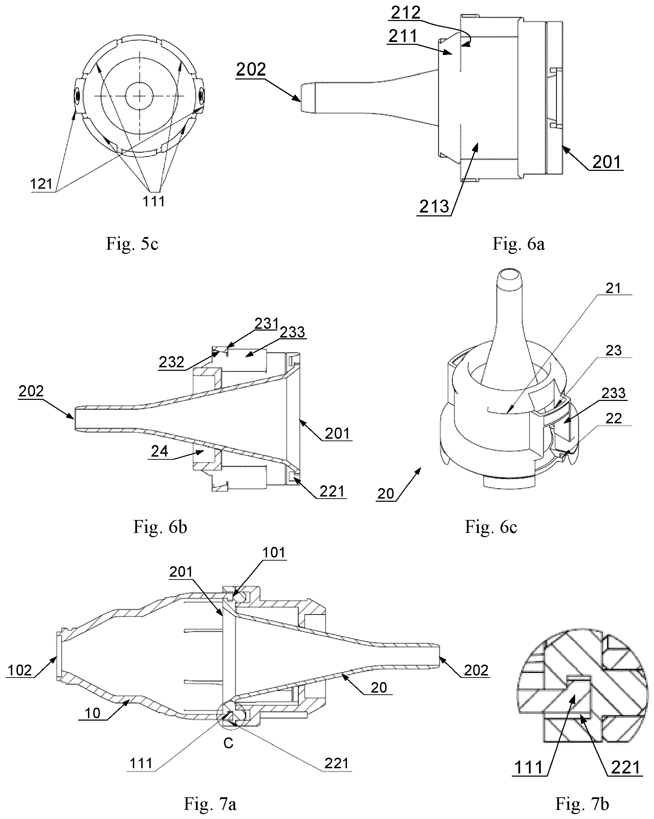

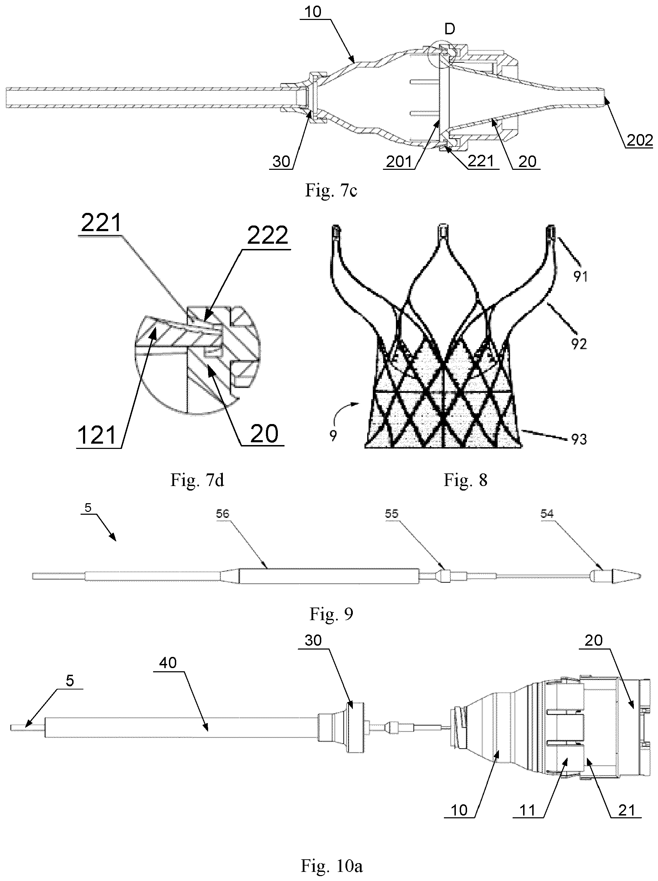

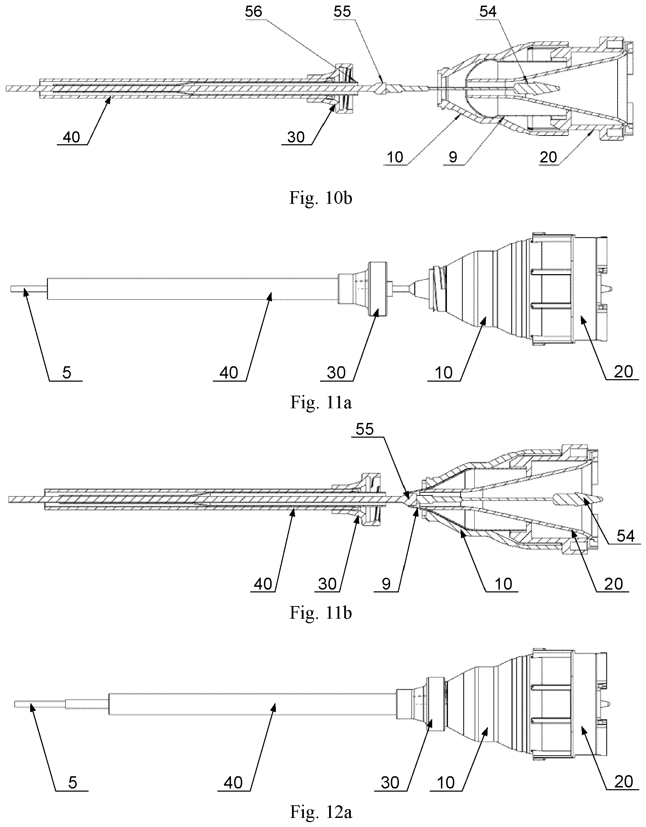

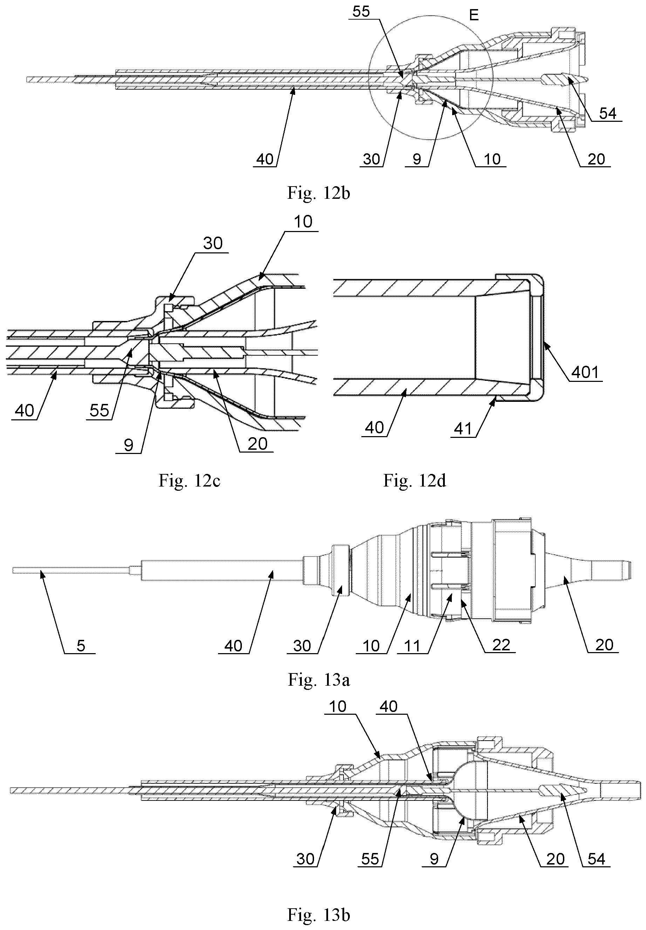

Reference is now made to Figs. 1 to 13b. Fig. 1 is a schematic illustration of an implant loading tool according to a first embodiment of the present invention. Fig. 2a is a schematic diagram illustrating a first snap fastener and a second snap fastener in snap engagement with each other according to the first embodiment of the present invention. Fig. 2b is a schematic cross-sectional view of Fig. 2a taken along an axial plane. Fig. 2c is an enlarged view of portion A of Fig. 2b. Fig. 3a is a schematic diagram illustrating a fourth snap fastener and a fifth snap fastener in snap engagement with each other according to the first embodiment of the present invention. Fig. 3b is a schematic cross-sectional view of Fig. 3a taken along an axial plane. Fig. 3c is an enlarged view of portion B of Fig. 3b. Fig. 4 is a schematic cross-sectional view taken along an axial plane, showing how a securing member, a capsule guide tube and an outflow cone are coupled together in accordance with the first embodiment of the present invention. Fig. 5a is a schematic illustration of the outflow cone according to the first embodiment of the present invention. Fig. 5b is a schematic cross-sectional view of the outflow cone of Fig. 5a taken along an axial plane. Fig. 5c schematically illustrates the outflow cone of Fig. 5a viewed in the direction from a first site toward a second site. Fig. 6a is a schematic illustration of an outflow cone according to the first embodiment of the present invention. Fig. 6b is a schematic cross-sectional view of the inflow cone of Fig. 6a taken along an axial plane. Fig. 6c is a perspective view of the inflow cone of Fig. 6a. Fig. 7a is a schematic diagram illustrating the first snap fastener and a third snap fastener in snap engagement with each other according to the first embodiment of the present invention. Fig. 7b is an enlarged view of portion C of Fig. 7a. Fig. 7c is a schematic diagram illustrating a side wall of a snap pocket according to the first embodiment of the present invention. Fig. 7d is an enlarged view of portion D of Fig. 7c. Fig. 8 is a schematic illustration of an implant according to the first embodiment of the present invention. Fig. 9 is a schematic illustration of a delivery device according to the first embodiment of the present invention. Fig. 10a is a schematic diagram illustrating a first step in a process of using the loading tool according to the first embodiment of the present invention. Fig. 10b is a schematic cross-sectional view of Fig. 10a taken along an axial plane. Fig. 11a is a schematic diagram illustrating a second step in the process of using the loading tool according to the first embodiment of the present invention. Fig. 11b is a schematic cross-sectional view of Fig. 11a taken along an axial plane. Fig. 12a is a schematic diagram illustrating a third step in the process of using the loading tool according to the first embodiment of the present invention. Fig. 12b is a schematic cross-sectional view of Fig. 12a taken along an axial plane. Fig. 12c is an enlarged view of portion E of Fig. 12b. Fig. 12d is a schematic partial cross-sectional view of the capsule guide tube of Fig. 12b taken along an axial plane. Fig. 13a is a schematic diagram illustrating a fourth step in the process of using the loading tool according to the first embodiment of the present invention. Fig. 13b is a schematic cross-sectional view of Fig. 13a taken along an axial plane.

As shown in Figs. 1 to 7d, the implant loading tool provided in the first embodiment of the present invention includes an outflow cone 10 and an inflow cone 20 detachably coupled to the outflow cone 10. The outflow cone 10 has axially opposite first 101 and second 102 sites and is configured to be coupled to the inflow cone 20 in the direction from the second site 102 to the first site 101. The inflow cone 20 has axially opposite third 201 and fourth 202 sites. The inflow cone 20 is configured to be inserted into the outflow cone 10 in a first direction so as to perform a first compression against an implant by applying a first compressive force to the implant. The inflow cone 20 is also configured to be detachably coupled to the outflow cone 10 in a second direction opposite to the first direction so as to perform a second compression against the implant by applying a second compressive force to the implant. The first direction is from the third site 201 to the fourth site 202, and the second direction is therefore from the fourth site 202 to the third site 201. Once the inflow cone 20 is coupled to the outflow cone 10 in the second direction, the inflow cone 20 is coaxial with the outflow cone 10 while being prevented from radial displacement and axial displacement relative to the outflow cone 10. Reference can be made to Figs. 10b and 11b for the first compression, which is an initial compression of the implant by the inflow cone 20 within the outflow cone 10. Reference can be made to Fig. 13b for the second compression, which involves compressing an end of the implant by the inflow cone 20 and then loading the implant into a delivery device 5.

Specifically, referring to Figs. 5a to 6c, in one exemplary embodiment, the outflow cone 10 comprises a first lumen extending therethrough, and the outflow cone 10 is stepwise tapered in the direction from the first site 101 to the second site 102. The inflow cone 20 comprises a receptacle 24, which is open toward the fourth site 202 and is configured to receive an end portion of the implant. The inflow cone 20 comprises a second lumen extending therethrough, and the second lumen is configured for passage of a part of the delivery device 5. The second lumen is taped in the direction from the third site 201 to the fourth site 202. In practice, since the implant is usually annular in shape before it is loaded in the delivery device 5, the receptacle 24 is also configured to be annular to match the geometry of the end portion of the implant. Referring to Figs. 10b and 11b, during insertion of the inflow cone 20 into the outflow cone 10 in the first direction, as the first lumen of the outflow cone 10 is stepwise tapered in the first direction, a radial compression of the implant (i.e., the first compression) can be performed.

After the implant is loaded in part in the delivery device 5, the inflow cone 20 may be detached, flipped over and then reassembled with the outflow cone 10 in the second direction at the first site 101 of the outflow cone 10, while ensuring that the inflow cone 20 is maintained coaxial with the outflow cone 10 and prevented from radial displacement and axial displacement relative thereto. In this way, a side wall of the second lumen can be brought into abutment against an inflow section 93 of the implant, which prevents radial displacement and axial displacement of the implant. As a result, the inflow section 93 of the implant is compressed at one end so as to fit the second lumen, facilitating coaxial loading of the implant into the delivery device 5.

With this arrangement, first of all, the first compression of the implant is performed by both the outflow cone 10 and the inflow cone 20. The inflow cone 20 is then flipped over and coupled to the outflow cone 10, thereby performing the second compression. During the second compression, the inflow cone 20 and the outflow cone 10 are maintained in coaxiality with each other, with the inflow cone 20 being prevented from radial displacement and axial displacement relative to the outflow cone 10. In this way, the inflow cone 20 is cleverly used to compress the implant twice in opposite orientations, entailing a simpler loading tool structure. Moreover, as the inflow cone 20 is maintained in coaxiality with the outflow cone 10 during the second compression, coaxial crimping of the implant is ensured for process of the second compression. This ensures straightness throughout the entire operation, which enables an operator to more easily perform the second compression, reduces the operator's operational difficulty and makes the whole crimping process easier and more efficient. Optionally, at least one of the outflow cone 10 and the inflow cone 20 is provided with a snap fastener, which is configured to restrict the inflow cone 20 and maintain it in coaxiality with the outflow cone 10 during the application of the first or second compressive force.

Referring to Figs. 5a to 7b, in conjunction with Figs. 2a to 3c, preferably, the outflow cone 10 has a first snap fastener 11, and the inflow cone 20 has a second snap fastener 21 and a third snap fastener 22. When the inflow cone 20 is inserted into the outflow cone 10 along the first direction, the first snap fastener 11 will snap-engage with the second snap fastener 21, preventing radial displacement and axial displacement of the outflow cone 10 relative to the inflow cone 20 and performing the first compression against the implant. When the inflow cone 20 is detachably coupled to the outflow cone 10 along the second direction, the first snap fastener 11 will snap-engage with the third snap fastener 22, preventing radial displacement and axial displacement of the outflow cone 10 relative to the inflow cone 20 and performing the second compression against the implant.

In an alternative embodiment, the first snap fastener 11 includes two or more first snap elements 111 distributed circumferentially around the outflow cone 10, and the outflow cone 10 has multiple cutout slots 13 extending axially with each opening toward the first site 101. In the outflow cone 10, cutout slots 13are provided on both circumferential sides of each first snap element 111. Preferably, the cutout slots may have any shape and size, with a rectangular shape being more preferred. The cutout slot is preferred to have a length of 18-21 mm along an axis of the outflow cone 10 and a width of 2-4 mm. With this arrangement, both elasticity and strength requirements can be satisfied. More preferably, both the outflow cone 10 and the inflow cone 20 may be made of a transparent material, such as PC or PMMA.

Preferably, each of the first snap elements 111 has a first snap surface 112 facing the second site 102 and a first engagement surface 113 facing the interior of the outflow cone 10. The second snap fastener 21 has a first bevel 211 tapered inwardly toward the fourth site 202, second snap surfaces 212 facing the third site 201 and a second engagement surface 213 facing (radially) toward the exterior of the inflow cone 20. During the insertion of the inflow cone 20 into the first lumen, the first engagement surfaces 113 will come into abutment against the first bevel 211. Due to the presence of the cutout slots 13, free ends of the first snap elements 111 that are separated by the cutout slots 13 are pushed by the first bevel 211 and increasingly deflect (radially) outwardly until the first snap surfaces 112 pass over the first bevel 211 and the first engagement surfaces 113 come into abutment against the second engagement surface 213. As a result, the outflow cone 10 is prevented from radial displacement relative to the inflow cone 20. The first snap surfaces 112 are configured to abut against the second snap surfaces 212 to prevent the outflow cone 10 from axial displacement toward the second site 102 relative to the inflow cone 20. The first snap fastener 11 will be considered to snap engage with the second snap fastener 21 when the first snap surfaces 112 come into abutment against the second snap surfaces 212. It is to be noted that, according to the present embodiment, axial displacement of the outflow cone 10 toward the first site 101 relative to the inflow cone 20 is not prevented. That is, with respect to the inflow cone 20, the outflow cone 10 is brought in coaxiality and prevented from displacement in one axial direction. When the inflow cone 20 is further advanced toward the second site 102 of the outflow cone 10, the first snap fastener 11 will be separated from the second snap fastener 21. In particular, the separation of the first snap fastener 11 from the second snap fastener 21 means that the first snap surfaces 112 is released from the abutment with the second snap surfaces 212 while the first engagement surfaces 113 are still in abutment with the second engagement surface 213. That is, the separation of the first snap fastener 11 from the second snap fastener 21 means that the inflow cone 20 is advanced further into the first lumen toward the second site 102 while relative radial displacement of the outflow cone 10 and inflow cone 20 is still prevented by the abutment of the first engagement surfaces 113 with the second engagement surface 213. With this arrangement, the outflow cone 10 and inflow cone 20 can be firmly maintained in coaxiality, facilitating the insertion of a conical head in the delivery device 5 and hence further compression of the implant. It is to be noted that although the first snap fastener 11 and the second snap fastener 21 prevent relative axial displacement of the outflow cone 10 and the inflow cone 20 by the abutment of the first snap surfaces 112 with the second snap surfaces 212, in some other embodiments, the first snap fastener 11 and the second snap fastener 21 may alternatively to prevent relative axial displacement of the outflow cone 10 and the inflow cone 20 by another form of engagement, such as that based on a friction fit or on recess(es) and protrusion(s). Preferably, the first snap surfaces 112 are distributed circumferentially around an axis of the outflow cone 10, and the second snap surfaces 212 are distributed circumferentially around an axis of the inflow cone 20. When the first snap fastener 11 snap engages with the second snap fastener 21, the outflow cone 10 and the inflow cone 20 are maintained in coaxiality with each other.

Further, the outflow cone 10 has a fourth snap fastener 12, and the inflow cone 20 has a fifth snap fastener 23. After the first snap fastener 11 snap engages with the second snap fastener 21, if the inflow cone 20 is inserted deeper into the outflow cone 10 in the first direction, the fourth snap fastener 12 will snap engage with the fifth snap fastener 23. As a result, the outflow cone 10 is prevented from radial displacement, axial displacement and circumferential rotation relative to the inflow cone 20, allowing a third compression of the implant to be conducted.

In an alternative embodiment, the fourth snap fastener 12 includes two or more second snap elements 121 distributed circumferentially around the outflow cone 10. In the outflow cone 10, cutout slots 13 are provided on both circumferential sides of each second snap element 121. It is to be noted that some of the cutout slots 13 on both sides of the second snap elements 121 may be the same as some of the cutout slots 13 on both sides of the first snap elements 111. That is, it may be the case that there is only one cutout slot 13 between a second snap element 121 and a first snap element 111. Optionally, the second snap elements 121 may be active snap elements, preferably elastic snap elements. The second snap elements 121 are not limited to having any particular shape. Preferably, the fourth snap fastener 12 is not limited to having any particular number of second snap elements 121, as long as it is ensured that relative circumferential and axial displacement of the outflow cone 10 and the inflow cone 20 is prevented when they engage with each other. As shown in Fig. 5c, preferably, there are two second snap elements 121, which are arranged in axial symmetry in the circumferential direction of the outflow cone 10. Optionally, for easy operation, exterior surfaces of the second snap elements 121 are marked in the same way as exterior surfaces of the fifth snap fastener 23.

Preferably, each of the second snap elements 121 has a second bevel 122 tapered inwardly toward the first site 101 and a third snap surface 123 facing the second site 102. The fifth snap fastener 23 has fourth snap surfaces 231 facing the fourth site 202 and first stop surfaces 232 facing the interior of the inflow cone 20. After the first snap fastener 11 snap engages with the second snap fastener 21, when the inflow cone 20 is advanced in the first direction deeper into the outflow cone 10, the first stop surfaces 232 will come into abutment against the inwardly-tapered second bevels 122 and radially push the second bevels 122 toward the interior of the outflow cone 10. As a result, free ends of the second snap elements 121 that are separated by the cutout slots 13 are pushed and increasingly deflect toward the interior of the outflow cone 10 until the third snap surfaces 123 pass over the first stop surfaces 232. Upon this happening, the free ends of the second snap elements 121 rebound outwardly and an external side wall of the outflow cone 10 comes into abutment against the first stop surfaces 232, preventing radial displacement of the outflow cone 10 relative to the inflow cone 20. At the same time, the third snap surfaces 123 abut against the fourth snap surfaces 231, preventing axial displacement of the outflow cone 10 toward the second site 102 relative to the inflow cone 20. The fourth snap fastener 12 will be considered to snap engage with the fifth snap fastener 23 when the third snap surfaces 123 come into abutment against the fourth snap surfaces 231. Preferably, the outflow cone 10 and the inflow cone 20 are kept coaxial with each other upon snap engagement of the fourth snap fastener 12 with the fifth snap fastener 23.

The fifth snap fastener 23 also has two second stop surfaces 233 arranged in opposition to each other along circumference of the inflow cone 20. A distance between the two second stop surfaces 233 matches an arc width of the specific second snap element 121 between the respective adjacent cutout slots 13. That is, the distance between the two second stop surfaces 233 is equal to or slightly greater than the arc width of the second snap element 121. The second stop surfaces 233 are configured to abut against respective radial side faces of the second snap element 121 to prevent circumferential rotation of the outflow cone 10 relative to the inflow cone 20. As shown in Fig. 3a, once the fourth snap fastener 12 snap engages with the fifth snap fastener 23, the two radial side faces of each second snap element 121 are in abutment with the respective two second stop surfaces 233 of the fifth snap fastener 23.

Referring to Figs. 11b and 12b, once the fourth snap fastener 12 snap engages with the fifth snap fastener 23, the outflow cone 10 and the inflow cone 20 are prevented from relative radial displacement, axial displacement and circumferential rotation and therefore stationary with respect to each other. This can facilitate the third compression of the implant by avoiding it from being twisted, tilted or worn during the compression.

Preferably, a maximum axial distance from the first snap fastener 11 to the second site 102 is not smaller than a maximum axial distance from the fourth snap fastener 12 to the second site 102. With this arrangement, the fourth snap fastener 12 does not extend beyond the first snap fastener 11 in the direction toward the first site 101. This not only can significantly reduce the size of the outflow cone 10 and thereby facilitate packaging, sterilization and other processing, but also leads to a reduction in cost.

Referring to Figs. 7a to 7d, in conjunction with Figs. 13a and 13b, the third snap fastener 22 preferably includes snap pockets 221, a cross-sectional shape of snap the pockets 221 along the axis of the inflow cone 20 matches a cross-sectional shape of the first snap elements 111 along the axis of the inflow cone 20. After the third compression of the implant is accomplished by the fourth snap fastener 12 snap engaging with the fifth snap fastener 23, the inflow cone 20 may be detached from the outflow cone 10 and flipped over so as to be tapered in the second direction, and the third snap fastener 22 of the inflow cone 20 may be then assembled with the first snap fastener 11 of the outflow cone 10. Specifically, the first snap elements 111 may snap engage in the snap pockets 221 and thus be prevented from radial and axial displacement by the snap pockets 221. As a result, the inflow cone 20 is prevented from radial and axial displacement relative to the outflow cone 10. Further, the second lumen of the inflow cone 20 comprises a location where the inflow section 93 of the implant is to be positioned. Preferably, the snap pockets 221 are distributed circumferentially around the axis of the inflow cone 20 so that the outflow cone 10 and the inflow cone 20 are brought into coaxiality with each other upon the snap engagement of the first snap elements 111 with the snap pockets 221.

It is to be noted that the matching of the cross-sectional shape of the snap pockets 221 along the axis of the inflow cone 20 with the cross-sectional shape of the first snap elements 111 along the axis of the inflow cone 20 is not limited to the scenario where the axial cross-sectional shape of the snap pockets 221 is identical to that of the first snap elements 111. The snap pockets 221 may be either slightly larger than the first snap elements 111, or equally sized to the first snap elements 111. It is also possible that the axial cross-sectional shape of the snap pockets 221 can envelop the axial cross-sectional shape of the first snap elements 111. In some embodiments, the axial cross-sectional shape of the snap pockets 221 resembles the letter "L", "T" or "J", etc., and the first snap elements 111 can be completely accommodated within the snap pockets 221, with their corners or edges abutting against the snap pockets 221. In this way, the first snap elements 111 are prevented from displacement by the snap pockets 221. Preferably, the number of the snap pockets 221 is not less than the number of the first snap elements 111. For example, the number of the snap pockets 221 may be two, three or four. Through providing the third snap fastener 22, the first snap fastener 11 is cleverly reused to entail a simpler loading tool structure. In addition to the snap engagement of the first snap fastener 11 and the second snap fastener 21, and the snap engagement of the fourth snap fastener 12 and the fifth snap fastener 23, a third snap engagement can be established, which ensures coaxiality of the loading tool and the implant.

Preferably, the snap pockets 221 are spaced circumferentially around the inflow cone 20 so that adjacent ones of them are spaced apart by a spacing area. Such spacing areas may be in the form of notches distributed circumferentially around the inflow cone 20, a circumferential length of the spacing areas is not smaller than a circumferential length of the first snap elements 111 measured with respect to the outflow cone 10. The spacing areas are configured so that the first snap elements 111 can be inserted therein in the direction toward the fourth site 202 and come into snap engagement with the snap pockets 221 as a result of a circumferential rotation caused by rotating the outflow cone 10 relative to the inflow cone 20 about the axis.

Referring to Figs. 7c and 7d, in order to facilitate easy snap engagement of the first snap elements 111 into the snap pockets 221, side walls 222 of the snap pockets 221 that face the interior of the inflow cone 20 preferably flare outwardly in the direction toward the third site 201. Figs. 7c and 7d show a configuration in which the second snap elements 121 are being inserted in the snap pockets 221. It would be appreciated that the first snap elements 111 are located in the spacing areas in this configuration. From this configuration, rotating the outflow cone 10 relative to the inflow cone 20 can cause the first snap elements 111 to circumferentially move into snap engagement with the snap pockets 221. At the same time, the second snap elements 121 leave the snap pockets 221 and enter the spacing areas.

Referring to Figs. 4 and 10a to 13b, the implant loading tool is preferred to further include a securing member 30 comprising an internal bore axially extending therethrough. The securing member 30 is configured to be detachably attached to the second site 102. When the securing member 30 is attached to the second site 102, the internal bore is coaxial with the outflow cone 10 and the securing member 30 is prevented from radial displacement and axial displacement relative to the outflow cone 10. In one embodiment, the securing member 30 comprises an internal bore axially extending therethrough, which allows passage of the delivery device 5 and radially restricts to some extent the delivery device 5. When the securing member 30 is assembled with the outflow cone 10 at the second site 102, the internal bore is coaxial with the outflow cone 10, ensuring coaxiality of the delivery device 5 with the outflow cone 10. Optionally, the securing member 30 may be attached to the outflow cone 10 by threads, snap means or a pin. Preferably, the securing member 30 is threadedly coupled to the outflow cone 10, because such threaded coupling can ensure coaxiality of the securing member 30 with the outflow cone 10.

The implant loading tool may further include a capsule guide tube 40 configured for detachable insertion in the internal bore. When the capsule guide tube 40 is coupled to the securing member 30, the capsule guide tube 40 is coaxial with the internal bore and prevented from radial displacement and axial displacement relative to the securing member 30. Optionally, an inner diameter of the internal bore may match an outer diameter of the capsule guide tube 40, the capsule guide tube 40 is inserted in the internal bore and is prevented by the internal bore from radial displacement relative to the securing member 30. Through providing the capsule guide tube 40 and the securing member 30, robust coupling of the inflow cone 10, the outflow cone 20, the securing member 30 and the capsule guide tube 40 with respect to the axial direction can be achieved, and hence the coaxiality among the four can be achieved. This can reduce the difficulty of crimping and hence related requirements on the operator.

In one exemplary embodiment, the capsule guide tube 40 has axially opposite fifth 401 and sixth 402 sites and is configured to be coupled to the securing member 30 in the direction from the sixth site 402 to the fifth site 401. The securing member 30 has axially opposite seventh 301 and eighth 302 sites and is configured to be coupled to the securing member 30 in the direction from the eighth site 302 to the seventh site 301. The capsule guide tube 40 has a third stop surface 41 facing the sixth site 402, and the securing member 30 has a fourth stop surface facing the seventh site 301. The third stop surface 41 is configured to abut against the fourth stop surface to prevent axial displacement of the capsule guide tube 40 toward the sixth site 402 relative to the securing member 30. Reference can be made to the patent publication No. CN209827106U for details in the configuration of the capsule guide tube 40, which is entirely incorporated herein by reference.

Referring to Figs. 9 to 13b, in this embodiment, there is also provided a medical device including the implant loading tool as defined above and a delivery device 5. The implant loading tool is used to cooperate with the delivery device 5 to load an implant into the delivery device 5. The implant is a compressible implant, such as a heart valve stent. The present invention is not limited to any particular shape or material of the implant, and any suitable existing implant is possible.

With the implant being a valve stent 9 as an example, how the implant loading tool is used and how the medical device is structured and operates will be explained below with reference to the accompanying drawings.

Referring to Fig. 8, the valve stent 9 includes an outflow section 92, an inflow section 93 and attachment projections 91. The above-discussed loading tool of this embodiment as discussed above is used to compress and crimp the valve stent 9 with the aid of the delivery device 5 and load the valve stent 9 into the delivery device 5. During subsequent use, the valve stent 9 is delivered in the crimped state by the delivery device 5 through a catheter to a target site in a patient's body and released there. After released, the valve stent 9 expands into a configuration as shown in Fig. 8.

Referring to Fig. 9, the delivery device 5 includes a conical head 54, an anchor 55, a sheath 56, a catheter and a handle (not shown). The conical head 54 is fixed to the anchor 55 which is received within the sheath 56. The anchor 55 is secured to the handle by a connecting member, and the handle can be manipulated to displace the sheath 56 so that the anchor 55 is exposed to allow the attachment projections 91 of the valve stent 9 to be attached thereto and thus serve as points of force application to the valve stent 9. In an optional embodiment, the anchor 55 has a circumferential groove, into which the attachment projections 91 can snap and thus attach to the anchor 55. After the attachment projections 91 snaps into the groove, the valve stent 9 is prevented from axial displacement relative to the anchor 55.

In Step 1, as shown in Figs. 10a and 10b, during use of the implant loading tool, the inflow section 93 of the valve stent 9 is first placed on the receptacle 24 of the inflow cone 20, and the outflow cone 10 is then pushed from the side of the outflow section 92 of the valve stent 9 in the second direction toward the inflow cone 20 (i.e., the inflow cone 2 is inserted into the first lumen), bringing the first snap fastener 11 of the outflow cone 10 into snap engagement with the second snap fastener 21 of the inflow cone 20. As a result, the outflow cone 10 is prevented from axial displacement relative to the inflow cone 20. At this time, since the valve stent 9 is stopped at one end by the receptacle 24, it is compressed by the first lumen of the outflow cone 10 for the first time. Moreover, because of the snap engagement of the first snap fastener 11 and the second snap fastener 21, the outflow cone 10 and the inflow cone 20 are in a first locked configuration where the outflow cone 10, the inflow cone 20 and the valve stent 9 are stably maintained coaxial, facilitating insertion of the conical head 54 and accomplishment of the first compression.

In Step 2, the conical head 54 is inserted into the second lumen from the fourth site 202 of the inflow cone 20 (at this time, the outflow cone 10 and the inflow cone 20 are in the first locked configuration with the valve stent 9 being interposed between the outflow cone 10 and the inflow cone 20) and the attachment projections 91 are substantially aligned with the anchor 55. The outflow cone 10 is then further advanced in the second direction toward the inflow cone 20 until the fourth snap fastener 12 snap engages with the fifth snap fastener 23, accomplishing a third compression. In this process, the outflow section 92 of the valve stent 9 is partially pushed out of the outflow cone 10 from the second site 102, while the outflow cone 10, the inflow cone 20 and the valve stent 9 remain fixed to one another, facilitating snap engagement of the attachment projections 91 in the groove on the anchor 55, as shown in Figs. 11a and 11b. After confirming that the attachment projections 91 have snapped into the groove on the anchor 55, the handle is manipulated to cause the sheath 56 to move toward the inflow section 93 (i.e., to the right as shown in the figures, toward the third site 201 of the inflow cone 20). As a result, the valve stent 9 starts moving into the sheath 56. During the first and third compressions of the valve stent 9, as the outflow cone 10 and the inflow cone 20 are immobilized relative to each other radially, circumferentially and axially, the valve stent 9 can be effectively avoided from being twisted, tilted or worn, allowing the compressions of the valve stent 9 to be proceed in a smooth fashion.

Referring to Figs. 12a and 12b, in Step 3, after the completion of the third compression of the valve stent 9, the securing member 30 is attached, for example, threadedly, to the outflow cone 10 so that the outflow cone 10, the inflow cone 20, the securing member 30 and the capsule guide tube 40 are secured together. After the operator confirms that the attachment projections 91 of the valve stent 9 do not dislodge from the groove on the anchor 55 in the delivery device 5, he/she may manipulate the handle to cause the sheath 56 to move toward a distal end of the delivery device 5 until approximately one third of the valve stent 9 enters the sheath 56. During the manipulation, the outflow cone 10 and the inflow cone 20 are secured to each other both circumferentially and axial, preventing the valve stent from being twisted, tilted or worn during compression and allowing the compression process to be proceed in a smooth fashion. With this done, initial loading of the valve stent 9 is accomplished.

Referring to Figs. 13a and 13b, in Step 4, after the completion of the initial loading of the valve stent 9, with the fourth snap fastener 12 being pressed, the inflow cone 20 is detached from the outflow cone 10, exposing the valve stent 9. The inflow cone 20 is flipped over, and the valve stent 9 is caused to move in the second direction toward the first site 101 of the outflow cone 10 so that its inflow section 93 comes into abutment against the side wall of the second lumen of the inflow cone 20. As a result, the inflow section 93 of the implant is compressed into conformity with the second lumen. Subsequently, the first snap fastener 11 is snap engaged with the third snap fastener 22, again coaxially securing the outflow cone 10, the inflow cone 20 and the valve stent 9 together. Afterward, the handle is manipulated to push the sheath 56 to move to the right as shown in the figures, and the loading tool 5 is simultaneously pushed to move to the right as shown in the figures, thereby causing the valve stent 9 to move deeper into the sheath 56. As a result, a second compression of the valve stent 9 is performed by the second lumen of the inflow cone 20, and the valve stent 9 is completely loaded into the sheath 56. With this done, the process of loading the valve stent 9 ends. During the second compression of the valve stent 9, the valve stent 9 is kept coaxial with the delivery device 5 by the second lumen of the inflow cone 20, solving the problem that existing loading tools requires manual intervention for coaxial crimping. Additionally, the present loading tool includes fewer components, has a simpler structure, can be fabricated at lower cost and is easy to operate.

### Embodiment 2

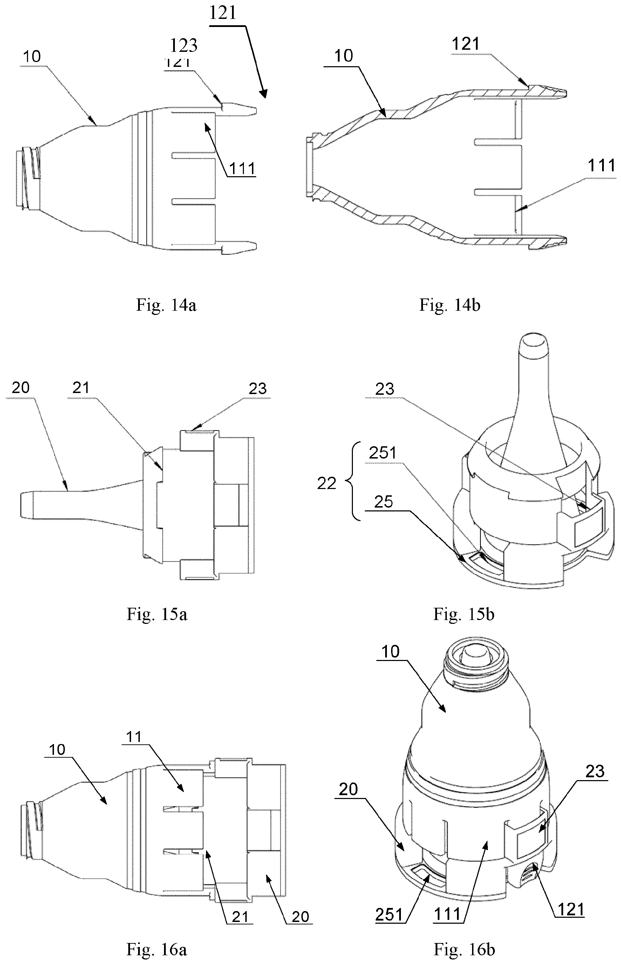

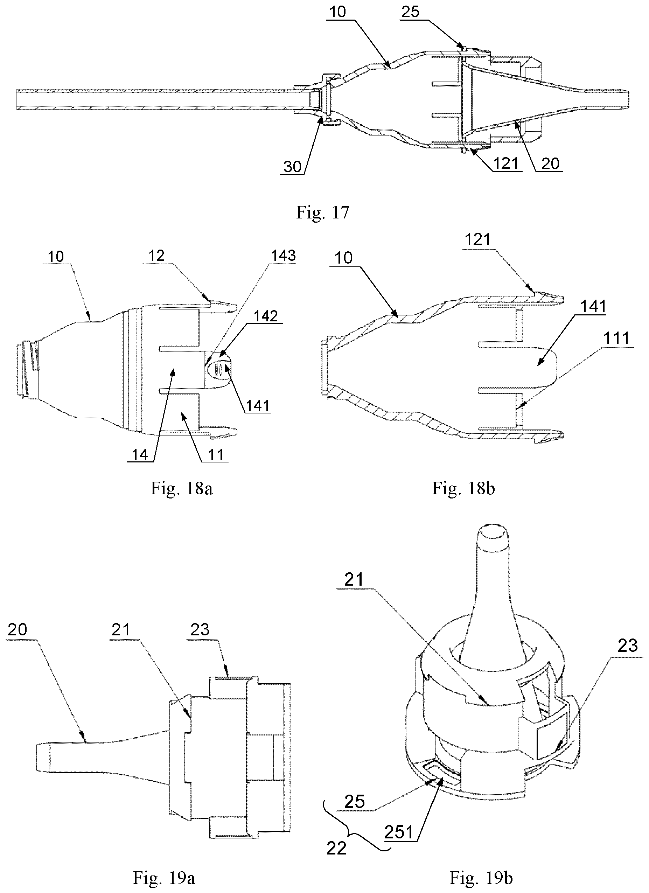

Reference is now made to Figs. 14a to 17. Fig. 14a is a schematic illustration of an outflow cone according to a second embodiment of the present invention. Fig. 14b is a schematic cross-sectional view of Fig. 14a taken along an axial plane. Fig. 15a is a schematic illustration of an inflow cone according to the second embodiment of the present invention. Fig. 15b is a schematic perspective view of Fig. 15a. Fig. 16a is a schematic diagram illustrating a first snap fastener and a second snap fastener in snap engagement with each other according to the second embodiment of the present invention. Fig. 16b is a schematic diagram illustrating a fourth snap fastener and a fifth snap fastener in snap engagement with each other according to the second embodiment of the present invention. Fig. 17 is a schematic diagram illustrating the fourth snap fastener and a third snap fastener in snap engagement with each other according to the second embodiment of the present invention.

In the second embodiment, an implant loading tool and a medical device are provided, which are similar to those of the first embodiment. Therefore, only the differences between them will be described below while the common features will not be described again.

As shown in Figs. 14a to 17, in use of the implant loading tool according to the second embodiment, after first and third compressions are accomplished, when the inflow cone 20 is detachably coupled to the outflow cone 10 along the second direction, the fourth snap fastener 12 will snap engage with the third snap fastener 22. In this way, the outflow cone 10 is prevented from radial displacement and axial displacement relative to the inflow cone 20, allowing a second compression of an implant to be conducted.

In one exemplary embodiment, the third snap fastener 22 includes a snap plate 25 arranged along the axial direction of the inflow cone 20. The snap plate 25 has snap openings 251 extending therethrough along the axial direction of the inflow cone 20. The second snap elements 121 are configured to snap engage into the snap openings 251, and the third snap surfaces 123 are configured to abut against a side of the snap plate 25 facing the fourth site 202, thereby preventing radial displacement and axial displacement of the inflow cone 20 relative to the outflow cone 10.

Preferably, the number of the snap openings 251 is not less than the number of the second snap elements 121.

In the second embodiment, the fourth snap fastener 12 is cleverly reused to perform the second compression against the implant through snap engaging the fourth snap fastener 12 with the third snap fastener 22. This entails a simpler loading tool structure, in which in addition to the snap engagement of the first snap fastener 11 and the second snap fastener 21 and the snap engagement of the fourth snap fastener 12 and the fifth snap fastener 23, a third snap engagement can be established, which ensures coaxiality of the loading tool and the implant.

In adaptation to the location of the snap plate 25, a maximum axial distance from the first snap fastener 11 to the second site 102 is preferably greater than a maximum axial distance from the fourth snap fastener 12 to the second site 102. In this way, when the inflow cone 20 is detachably coupled to the outflow cone 10 in the second direction, the second snap elements 121 snap engages with the snap plate 25 before the first snap elements 111 come into abutment against the inflow cone 20.

It is to be noted that the snap engagement of the fourth snap fastener 12 with the third snap fastener 22 according to the second embodiment may be combined with the snap engagement of the first snap fastener 11 and the third snap fastener 22 according to the first embodiment. In particular, the third snap fastener 22 may include both the snap pocket 221 and the snap plate 25, and after the first and third compressions are accomplished, when the inflow cone 20 is detachably coupled to the outflow cone 10 in the second direction, the first snap elements 111 comes into snap engagement with the snap pockets 221, and the second snap elements 121 come into snap engagement with the snap plate 25 at the same time.

### Embodiment 3

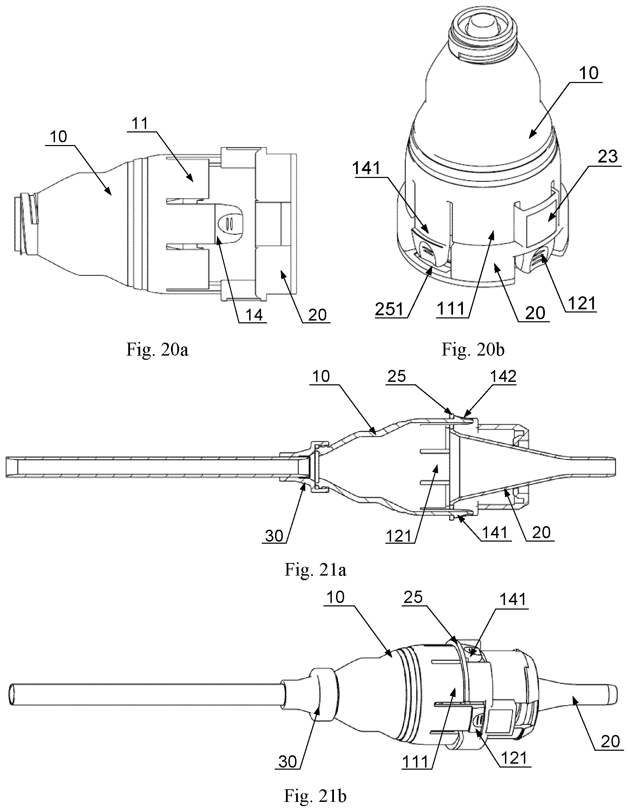

Reference is now made to Figs. 18a to 21b. Fig. 18a is a schematic illustration of an outflow cone according to a third embodiment of the present invention. Fig. 18b is a schematic cross-sectional view of Fig. 18a taken along an axial plane. Fig. 19a is a schematic illustration of an inflow cone according to the third embodiment of the present invention. Fig. 19b is a schematic perspective view of Fig. 19a. Fig. 20a is a schematic diagram illustrating a first snap fastener and a second snap fastener in snap engagement with each other according to the third embodiment of the present invention. Fig. 20b is a schematic diagram illustrating a fourth snap fastener and a fifth snap fastener in snap engagement with each other according to the third embodiment of the present invention. Fig. 21a is a schematic diagram illustrating the fourth snap fastener and a third snap fastener in snap engagement with each other according to the third embodiment of the present invention. Fig. 21b is a perspective view of the fourth snap fastener and the third snap fastener in snap engagement with each other according to the third embodiment of the present invention.

In the third embodiment, an implant loading tool and a medical device are provided, which are similar to those of the first embodiment. Therefore, only the differences between them will be described below, while the common features will not be described again.

As shown in Figs. 18a to 21b, in use of the implant loading tool according to the third embodiment, after first and third compressions are accomplished, instead of the first snap fastener 11 or the fourth snap fastener 12, a separate sixth snap fastener 14 provided on the outflow cone 10 is snap engaged with the third snap fastener 22.

Specifically, the outflow cone 10 is provided with the sixth snap fastener 14, when the inflow cone 20 is detachably coupled to the outflow cone 10 along the second direction, the sixth snap fastener 14 comes into snap engagement with the third snap fastener 22, preventing the outflow cone 10 from radial displacement and axial displacement relative to the inflow cone 20 and hence enabling a second compression of an implant to be conducted.

In one exemplary embodiment, the sixth snap fastener 14 includes two or more third snap elements 141, and the outflow cone 20 has multiple cutout slots 13 extending axially with each opening toward the first site 101. The third snap elements 141 are distributed circumferentially around the outflow cone 20 and cutout slots 13 are provided on both two circumferential sides of each third snap element 141. Each of the third snap elements 141 has a third bevel 142 facing the exterior of the outflow cone 10 and a fifth snap surface 143 facing the second site 102. The third bevel 142 is inwardly tapered toward the first site 101. It is to be noted that some of the cutout slots 13 on both sides of the third snap elements 141 may be the same as some of the cutout slots 13 on both sides of the first snap elements 111 or the second snap elements 121. That is, it may be the case that there is only one cutout slot 13 between a third snap element 141 and a second snap element 121 or a first snap element 111.

Preferably, the number of the third snap elements 141 in the sixth snap fastener 14 is not limited to any specific number, as long as it can be ensured that the outflow cone 10 and the inflow cone 20 are coupled together so as to be fixed in position with respect to each other. Preferably, there are two third snap elements 141 arranged at axisymmetric circumferential locations of the outflow cone 10.

### Embodiment 4

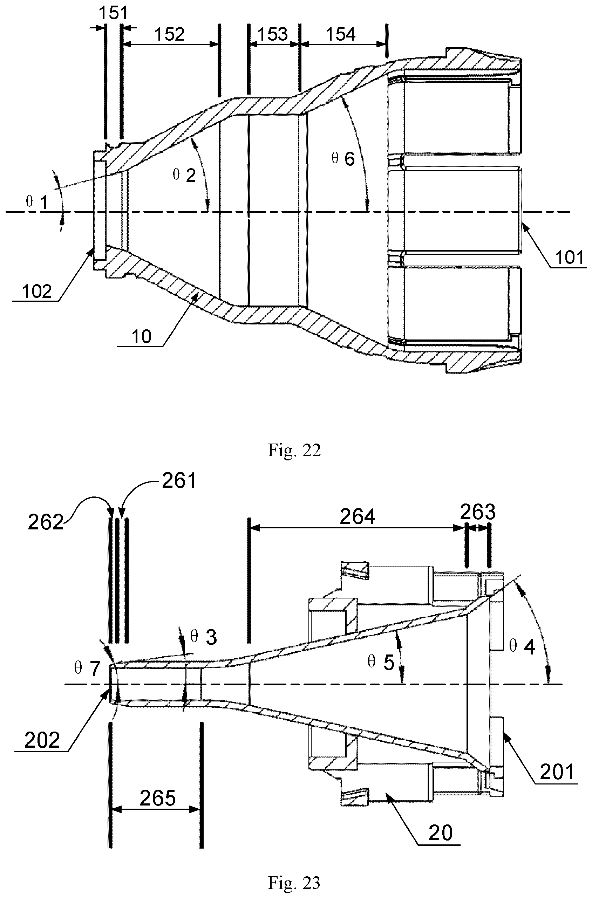

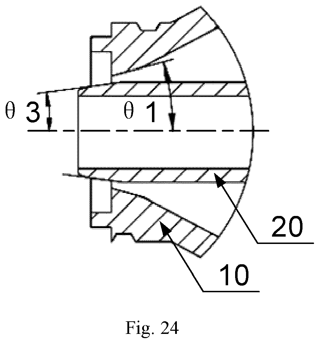

Reference is now made to Figs. 22 to 24. Fig. 22 is a schematic cross-sectional view of an outflow cone according to a fourth embodiment of the present invention taken along an axial plane. Fig. 23 is a schematic cross-sectional view of an inflow cone according to the fourth embodiment of the present invention taken along an axial plane. Fig. 24 is a schematic partial cross-sectional view of the outflow cone and the inflow cone being coupled to each other according to the fourth embodiment of the present invention taken along an axial plane.

In the fourth embodiment, an implant loading tool and a medical device are provided, which are similar to those of the first embodiment. Therefore, only the differences between them will be described below, while the common features will not be described again.

In the fourth embodiment, first, second and third compressions of an implant can be performed by the outflow cone 10 and the inflow cone 20 by the same steps as in the first embodiment, and reference can be made to the description in the first embodiment regarding these steps in the process of using the loading tool. In the fourth embodiment, a detailed description is set forth regarding structural details of the outflow cone 10 and the inflow cone 20.

Referring to Figs. 22 to 24, in the outflow cone 10, the first lumen includes a first section 151 at the second site 102 in the form of a conical frustum flaring in the direction from the second site 102 to the first site 101. A side wall of the first section 151 forms a first angle θ1 with the axis of the outflow cone 10. An outer circumference of the inflow cone 20 includes a second section 261 configured to fit with the first section 151. The second section 261 is in the form of a conical frustum flaring in the direction from the fourth site 202 to the third site 201. A side wall of the second section 261 forms, with the axis of the inflow cone 20, a second angle θ3 that is smaller than the first angle θ1.
when inserting the inflow cone 20 into the first lumen of the outflow cone 10 along the first direction, the fourth snap fastener 12 snap engages with the fifth snap fastener 23, allowing a third compression of the valve stent 9 to be conducted. At this time, the fourth site 202 of the inflow cone 20 protrudes out of the first lumen and is beyond the second site of the outflow cone 10. In addition, the second section 261 is aligned with and adjacent to the first section 151, and the two abut against the valve stent 9 respectively from the inner and outer sides of the valve stent 9. That is, the second section 261 and the first section 151 can be considered as fitting with each other.

As can be understood with additional reference to Fig. 11b, in this configuration, a contour shape of the valve stent 9 is determined by outer contours of portions of the first lumen of the outflow cone 10 and the inflow cone 20 in proximity to the fourth site 202. Moreover, the attachment projections 91 of the valve stent 9 extend in the first direction out of the second site of the outflow cone 10 and engages with the anchor 55 in the delivery device 5.

It will be appreciated that the direction of extension of the attachment projections 91 depends on the geometries of a portion of the first lumen proximal to the second site 102 and of a portion of the inflow cone 20 proximal to the fourth site 202. After research, the inventors found that, when the second angle θ3 is smaller than the first angle θ1, the attachment projections 91 of the crimped valve stent 9 will be oriented at a suitable angle, which allows the attachment projections 91 to effectively, firmly engage with the anchor 55 in the delivery system 5 without dislodgement therefrom, facilitating the next compression (i.e., a second compression). Moreover, since the first section 151 and the second section 261 are both conical frusta, the valve stent 9 can be always maintained in a steady state, and it can be ensured that the valve stent 9 is regularly compressed throughout its circumference and will not be compressed into any irregular shape.

The first lumen further includes a third section 152 adjacent to the first section 151. The third section 152 is on the side of the first section 151 proximal to the first site 101 and is in the form of a conical frustum flaring in the direction from the second site 102 to the first site 101. The junction of the first section 151 and the third section 152 is only inwardly convex. A side wall of the third section 152 forms a third angle θ2 with the axis of the outflow cone 10, and the first angle θ1 is smaller than the third angle θ2. It is to be noted that, by the phrase "the junction of the first section 151 and the third section 152 is only inwardly convex", it is intended to mean that there is no inward concavity toward the interior of the outflow cone 10 at the junction of the first section 151 and the third section 152. In one embodiment, both the first section 151 and the third section 152 are conical frusta and can be directly joined together. That is, a lower base of the first section 151 (i.e., the base proximal to the first site 101) and an upper base of the third section 152 (i.e., the base proximal to the second site 102) coincide in the axial direction of the outflow cone 10. To this end, the lower base of the first section 151 and the upper base of the third section 152 are equally sized and arranged in coincidence with each other. With this arrangement, upon joining of the first section 151 and the third section 152, there will be an angle between their side walls, which is only convex toward the interior of the outflow cone 10. In some other embodiments, the first section 151 and the third section 152 may be joined together by an additional transition section, for example, by a smooth curv that is inwardly convex or multiple polylines that are convex toward the interior of the outflow cone 10. In these cases, the first section 151 and the third section 152 are still considered to be adjacent to each other. However, it will be appreciated that there is no curve or polylines concavity toward the interior of the outflow cone 10. Configuring the junction of the first section 151 and the third section 152 to be only inwardly convex can ensure that the valve stent 9 will not be stuck in the first lumen. The phases "only inwardly convex" and "only outwardly convex" that may be used hereinafter to describe relationships between other sections can be understood with reference to the description regarding the junction of the first section 151 and the third section 152.

Optionally, the first lumen may further include adjacent sixth 153 and seventh 154 sections. The sixth section 153 is located on the side of the third section 152 proximal to the first site 101, and the seventh section 154 is located on the side of the sixth section 153 proximal to the first site 101. The sixth section 153 is cylindrical, and the seventh section 154 is in the form of a conical frustum flaring in the direction from the second site 102 to the first site 101. Both the junctions of the sixth section 153 and the third section 152, and of the seventh section 154 and the sixth section 153 are only inwardly convex.

Since the first lumen serves mainly to compress the valve stent 9, it is designed with the various sections with different inner diameters. Through providing the sixth section 153 and the seventh section 154, smooth transitioning of the valve stent 9 can be achieved. The four-section design of the first lumen is intended to compress and secure the valve stent 9 in a section-by-section manner. This not only allows both easier application of forces by a loading operator but also reduce loading complexity. In one exemplary embodiment, a maximum diameter of the seventh section 154 (i.e., the diameter at its base proximal to the first site 101) is desired to be greater than a maximum outer diameter of the valve stent 9 when the latter is in an expanded configuration in order to allow the valve stent 9 to enter the seventh section 154 without being hindered and then to be compressed therein.

Preferably, a side wall of the seventh section 154 forms with the axis of the outflow cone 10 a sixth angle θ6 in the range of 25°-30°. According to mechanical calculations and experimental validations conducted by the inventors, when the sixth angle θ6 is in the range of 25°-30°, the valve stent 9 can be compressed appropriately. The sixth section 153 is in a form of a cylindrical, and its side wall is parallel to the axis of the outflow cone 10. Preferably, a diameter of the sixth section 153 is about three-quarters of the maximum outer diameter of the valve stent 9 when the latter is in said expanded configuration. With this arrangement, the inflow section 93 can be held firmly to allow the outflow cone 10, the valve stent 9 and the inflow cone 20 to be maintained stationary relative to one another. Moreover, only the outflow section 92 will be compressed, with the inflow section 93 being not affected at all.

Optionally, the third angle θ2 may range from 25° to 30°. The third section 152 is configured to additionally compress the valve stent 9. Likewise, according to mechanical calculations and experimental validations, when the third angle θ2 is in the range of 25-30°, the valve stent 9 can be compressed appropriately, making the operator's force application easier and enabling stepwise compression. In a preferred embodiment, the third angle θ2 and the sixth angle θ6 may be configured to be equal.

Preferably, the second angle θ3 is in the range of 6.5° -8.5° and the first angle θ1 is in the range of 12.5°-17.5°. More preferably, the first angle θ1 is 15°. According to finite element simulations and experimental studies, this enables the attachment projections 91 of the valve stent 9 to be inwardly anchored to an extent that is not too tight to cause excessive deformation, as shown in Fig. 24. The special tapered designs of the first section 151 and the second section 261 enable the valve stent 9 to be always maintained in a steady state, and it can be ensured that the valve stent 9 is regularly compressed throughout its circumference and will not be compressed into any irregular shape.

Optionally, the outer circumference of the inflow cone 20 may further include an eighth section 262 located at the fourth site 202 and adjacent to the second section 261. A side wall of the eighth section 262 forms a seventh angle θ7 with the axis of the inflow cone 20, which is greater than the second angle θ3. The junction of the eighth section 262 and the second section 261 is only outwardly convex. Compared with the second section 261, the eighth section 262 is more steeply inwardly tapered in the first direction in order to avoid the valve stent 9 from being hindered or scratched. Preferably, the seventh angle θ7 ranges from 15° to 20°.

Referring to Fig. 23, in the outflow cone 20, the second lumen of the inflow cone 20 includes adjacent fourth 263 and fifth 264 sections. The fourth section 263 is located at the third site 201, and the fifth section 264 is located on the side of the fourth section 263 proximal to the fourth site 202. The fourth section 263 and the fifth section 264 are both in the form of conical frustums flaring in the direction from the fourth site 202 to the third site 201. The junction of the fourth section 263 and the fifth section 264 is only inwardly convex. A side wall of the fourth section 263 forms a fourth angle θ4 with the axis of the inflow cone 20, and a side wall of the fifth section 264 forms a fifth angle θ5 with the axis of the inflow cone 20. The fourth angle θ4 is greater than the fifth angle θ5.

After a third compression of the valve stent 9 is accomplished, the inflow cone 20 is assembled along the second direction with the first site 101 of the outflow cone 10, allowing a second compression of the valve stent 9 to be conducted. The second compression relies mainly on limitation of movement of one site of the inflow section 93 of the valve stent 9 by the second lumen of the inflow cone 20 and cooperation with the delivery device 5. Optionally, the second lumen may be designed with multiple sections with different inner diameters, which enable smooth transitioning of the valve stent 9. In an alternative embodiment, the second lumen further includes a ninth section 265 joined to one side of the fifth section 264 proximal to the fourth site 202. The ninth section 265 is a cylindrical section. In the final stage of the second compression, the valve stent 9 will be pushed into the ninth section 265 and undergo the final radial compression therein. Optionally, the ninth section 265 is smoothly joined to the fifth section 264 sand their junction is only inwardly convex. A diameter of the ninth section 265 is configured to be slightly greater than an outer diameter of the valve stent 9 when the latter is in a crimped configuration. An axial length of the ninth section 265 is preferred to be greater than 25mm to allow the valve stent 9 to be stable therein.

In the example shown in Fig. 23, the second lumen adopts a three-section design, and a maximum diameter of the fourth section 263 (i.e., the diameter at its base proximal to the third site 201) is desired to be greater than a maximum outer diameter of the inflow section 93 of the valve stent 9 after the latter has undergone the third compression. The fifth section 264 is configured to further compress the valve stent 9. As the fifth section 264 has a relatively large axial length and the fifth angle θ5 is relatively small, the valve stent 9 can be easily compressed from a relatively large diameter to a relatively small diameter. In this way, a relatively significant diameter change of the valve stent 9 can be achieved with less effort exerted by the operator.

Preferably, the fourth angle θ4 ranges from 25° to 30°, and the fifth angle θ5 ranges from 10° to 20°. With special angle designs for the fourth angle θ4 and the fifth angle θ5, the inner side wall of the fifth section 264 of the second lumen provides a gently tapered long compression path enabling gradual compression of the valve stent 9. This can better ensure that the valve stent 9 is progressively stressed while it is being crimped, avoiding it from forming an irregular shape in the crimped configuration.

As experimentally verified by the inventors, when the implant loading tool provided in the fourth embodiment is used to compress the valve stent 9, the attachment projections 91 can completely fit in the groove of the anchor 55. All the attachment projections 91 closely engage with the anchor 55, and the stent is crimped into a regular shape, which further facilitates balanced crimping of the valve stent 9 and enables smooth entry of the valve stent 9 into the sheath 56.

In summary, the present invention provides an implant loading tool and a medical device. The implant loading tool includes an outflow cone and an inflow cone configured to be detachably coupled to the outflow cone. The inflow cone is configured to be inserted into the outflow cone along a first direction to perform a first compression against an implant. The inflow cone is also configured to be detachably coupled to the outflow cone in a second direction opposite to the first direction, thereby performing a second compression against the implant. When the inflow cone is coupled to the outflow cone along the second direction, the inflow cone is coaxial with the outflow cone and is prevented from radial displacement and axial displacement relative to the outflow cone. With this arrangement, performing the first compression against the implant by the outflow cone and the inflow cone, and the inflow cone is then flipped over and coupled to the outflow cone to perform the second compression against the implant. During the second compression, the inflow cone is kept coaxial with the outflow cone and prevented from radial displacement and axial displacement relative to outflow cone. In this way, the inflow cone is cleverly used to compress the implant twice in opposite directions, entailing a simpler loading tool structure and enabling the inflow cone and the outflow cone to be maintained in coaxiality during the second compression. This ensures coaxial crimping of the implant during the second compression and straightness throughout the entire operation. As a result, an operator is enabled to more easily perform the second compression, and the whole crimping process is made easier and more efficient.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. An implant loading tool, comprising an outflow cone and an inflow cone configured to be detachably coupled to the outflow cone, wherein the outflow cone has, along an axis thereof, a first site and a second site that is opposite to the first site, wherein the outflow cone is configured to be coupled to the inflow cone in a direction from the second site to the first site, and wherein the inflow cone has, along an axis thereof, a third site and a fourth site that is opposite to the third site,
wherein the inflow cone is configured to be inserted into the outflow cone in a first direction from the third site to the fourth site to perform a first compression against an implant, and wherein the inflow cone is further configured to be detachably coupled to the outflow cone in a second direction from the fourth site to the third site to perform a second compression against the implant,
wherein when the inflow cone is coupled to the outflow cone along the second direction, the inflow cone is coaxial with the outflow cone, and the inflow cone is prevented from radial displacement and axial displacement with respect to the outflow cone.

2. The implant loading tool according to claim 1, wherein: the outflow cone comprises a first snap fastener and a fourth snap fastener; and the inflow cone comprises a second snap fastener, a fifth snap fastener and a third snap fastener,
wherein when the inflow cone is inserted into the outflow cone along the first direction, the first snap fastener snap engages with the second snap fastener, thereby preventing the outflow cone from radial displacement and axial displacement with respect to the inflow cone and performing the first compression against the implant, wherein when the inflow cone is further inserted into the outflow cone along the first direction after the snap engagement of the first snap fastener with the second snap fastener, the fourth snap fastener snap engages with the fifth snap fastener, thereby preventing the outflow cone from radial displacement, axial displacement and circumferential rotation with respect to the inflow cone and performing a third compression against the implant,
wherein when the inflow cone is detachably coupled to the outflow cone in the second direction, the first snap fastener and/or the fourth snap fastener snap engages with the third snap fastener, thereby preventing the outflow cone from radial displacement and axial displacement with respect to the inflow cone and performing the second compression against the implant.

3. The implant loading tool according to claim 2, wherein the first snap fastener comprises at least two first snap elements, wherein the fourth snap fastener comprises at least two second snap elements, wherein the outflow cone comprises a plurality of cutout slots extending axially, the cutout slots being open at the first site, wherein the first snap elements and the second snap elements are distributed circumferentially around the outflow cone, wherein the cutout slots are arranged on both circumferential sides of each first snap element, and wherein the cutout slots are arranged on both circumferential sides of each second snap element.

4. The implant loading tool according to claim 3, wherein each first snap element has a first snap surface facing the second site and a first engagement surface facing an interior of the outflow cone; wherein the second snap fastener has a first bevel inwardly-tapered toward the fourth site, a second snap surface facing the third site and a second engagement surface facing an exterior of the inflow cone; wherein the first bevel is configured to abut against the first engagement surface and to push a free end of the first snap element to deflect outwardly; wherein the first engagement surface is configured to abut against the second engagement surface and thereby preventing the outflow cone from radial displacement with respect to the inflow cone; and wherein the first snap surface is configured to abut against the second snap surface and thereby preventing the outflow cone from axial displacement toward the second site with respect to the inflow cone.

5. The implant loading tool according to claim 4, wherein the third snap fastener comprises snap pockets, wherein a cross-sectional shape of the third snap fastener along an axis of the inflow cone matches a cross-sectional shape of the first snap element along an axis of the outflow cone, and wherein the first snap element is configured to snap engage into the corresponding snap pocket and thereby preventing the inflow cone from radial displacement and axial displacement with respect to the outflow cone.

6. The implant loading tool according to claim 5, wherein a spacing area is provided between adjacent snap pockets, wherein a circumferential length of the spacing area is not smaller than a circumferential length of the first snap element along the outflow cone, wherein the spacing area is configured for insertion of the first snap element therein toward the fourth site, and wherein the first snap elements are configured to circumferentially move into snap engagement with the snap pockets as a result of a relative circumferential rotation of the outflow cone and the inflow cone.

7. The implant loading tool according to claim 6, wherein a side wall of each snap pocket facing the interior of the inflow cone flares outwardly toward the third site.

8. The implant loading tool according to claim 3, wherein each second snap element has a second bevel facing the exterior of the outflow cone and a third snap surface facing the second site, wherein the second bevel is tapered inwardly toward the first site, wherein the fifth snap fastener has a fourth snap surface facing the fourth site, a first stop surface facing an interior of the inflow cone and two second stop surfaces arranged in opposition to each other along a circumference of the inflow cone, wherein the first stop surface is configured to abut against the second bevel so as to push a free end of the second snap element to deflect toward the interior of the outflow cone, wherein the first stop surface is configured to abut against an external side wall of the outflow cone and thereby preventing the outflow cone from radial displacement with respect to the inflow cone, wherein the third snap surface is configured to abut against the fourth snap surface and thereby preventing the outflow cone from axial displacement toward the second site with respect to the inflow cone, wherein the second stop surfaces are configured to abut against radial side faces of the second snap elements and thereby preventing the outflow cone from circumferential rotation with respect to the inflow cone.

9. The implant loading tool according to any one of claims 2 to 8, wherein a maximum axial distance from the first snap fastener to the second site is not smaller than a maximum axial distance from the fourth snap fastener to the second site.

10. The implant loading tool according to claim 9, wherein the third snap fastener comprises snap plates each arranged along an axial direction of the inflow cone, wherein each snap plate has a snap opening extending therethrough in the axial direction of the inflow cone, wherein the second snap element is configured to snap engage into the snap opening, and wherein the third snap surface of the second snap element facing the second site is configured to abut against a side of the snap plate facing the fourth site and thereby preventing the inflow cone from radial displacement and axial displacement with respect to the outflow cone.

11. The implant loading tool according to claim 1, wherein the outflow cone comprises a sixth snap fastener and the inflow cone comprises a third snap fastener,
when the inflow cone is detachably coupled to the outflow cone in the second direction, the sixth snap fastener snap engages with the third snap fastener, thereby preventing the outflow cone from radial displacement and axial displacement with respect to the inflow cone.

12. The implant loading tool according to claim 11, wherein the sixth snap fastener comprises at least two third snap elements and the outflow cone comprises a plurality of cutout slots extending axially, the cutout slots being open at the first site, wherein the third snap elements are distributed circumferentially around the outflow cone and the cutout slots are arranged on both circumferential sides of each third snap element, wherein each third snap element has a third bevel facing an exterior of the outflow cone and a fifth snap surface facing the second site, the third bevel inwardly tapered toward the first site.

13. The implant loading tool according to claim 12, wherein the third snap fastener comprises snap plates each arranged along an axial direction of the inflow cone, wherein each snap plate has a snap opening extending therethrough in the axial direction of the inflow cone, wherein the third snap element is configured to snap engage into the snap opening, and wherein the fifth snap surface is configured to abut against a side of the snap plate facing the fourth site and thereby preventing the inflow cone from radial displacement and axial displacement with respect to the outflow cone.

14. The implant loading tool according to claim 1, further comprising a securing member including an internal bore axially extending therethrough, wherein the securing member is configured to be detachably coupled to the second site; wherein when the securing member is coupled to the second site, the internal bore is coaxial with the outflow cone and the securing member is prevented from radial displacement and axial displacement with respect to the outflow cone.

15. The implant loading tool according to claim 14, wherein the securing member is coupled to the outflow cone by threads, snap means or a pin.

16. The implant loading tool according to claim 14, further comprising a capsule guide tube, wherein the capsule guide tube is configured to be detachably inserted in the internal bore, wherein when the capsule guide tube is coupled to the securing member, the capsule guide tube is coaxial with the internal bore and is prevented from radial displacement and axial displacement with respect to the securing member.

17. The implant loading tool according to claim 16, wherein an inner diameter of the internal bore matches an outer diameter of the capsule guide tube, and wherein the capsule guide tube is configured to be inserted in the internal bore and is prevented from radial displacement with respect to the securing member by the internal bore.

18. The implant loading tool according to claim 16, wherein the capsule guide tube has, along an axis thereof, a fifth site and a sixth site that is opposite to the fifth site and is configured to be coupled to the securing member in a direction from the sixth site to the fifth site, wherein the securing member has, along an axis thereof, a seventh site and an eighth site that is opposite to the seventh site, wherein the capsule guide tube is configured to be coupled to the securing member in a direction from the eighth site to the seventh site, wherein the capsule guide tube has a third stop surface facing the sixth site, and that the securing member has a fourth stop surface facing the seventh site, and wherein the third stop surface is configured to abut against the fourth stop surface and thereby preventing the capsule guide tube from axial displacement toward the sixth site with respect to the securing member.

19. A medical device, comprising the implant loading tool as defined in any one of claims 1 to 18 and a delivery device, wherein the loading tool is configured to work with the delivery device to load an implant into the delivery device.

20. An implant loading tool, comprising an outflow cone and an inflow cone configured to be detachably coupled to the outflow cone, wherein at least one of the outflow cone and the inflow cone is provided with a snap fastener, wherein the inflow cone has, along an axis thereof, a third site and a fourth site that is opposite to the third site, wherein when the inflow cone is inserted into the outflow cone in a first direction from the third site to the fourth site, the inflow cone is configure to apply a first compressive force to the implant, wherein when the inflow cone is detachably coupled to the outflow cone in a second direction from the fourth site to the third site, the inflow cone is configured to apply a second compressive force to the implant, and wherein during the application of the first or second compressive force, the snap fastener is configured to limit the inflow cone so as to bring the inflow cone into coaxiality with the outflow cone.
